# EUROPÄISCHE PATENTSCHRIFT

(11) **EP 0 417 603 B1**
(45) Veröffentlichungstag und Bekanntmachung des Hinweises auf die Patenterteilung: **17.01.2001**
(21) Anmeldenummer: 90116964.9
(22) Anmeldetag: 04.09.1990
(51) Int. Cl.: C07D 229/00, C08G 18/79

(54) **Verfahren zur Herstellung von Uretdiongruppen aufweisenden Polyisocyanaten**
Process for the preparation of urethdione group-containing polyisocyanates
Procédé de préparation de polyisocyanates contenant des groupes urétidinedione

(30) Priorität: 14.09.1989 DE 3930669
(43) Veröffentlichungstag der Anmeldung: 20.03.1991
(73) Patentinhaber: BASF Aktiengesellschaft, 67063 Ludwigshafen (DE)
(72) Erfinder: Bruchmann, Bernd, Dr., D-6700 Ludwigshafen (DE)

(56) Entgegenhaltungen:
- EP-A- 0 045 995
- EP-A- 0 166 172
- DE-A- 1 643 170

## Beschreibung

Die Erfindung betrifft ein Verfahren zur Herstellung von Uretdiongruppen aufweisenden Polyisocyanaten durch Reaktion von monomeren aromatischen Diisocyanaten in Anwesenheit eines Katalysators.

Es ist grundsätzlich bekannt, Uretdione durch Umsetzung von Isocyanaten in Gegenwart bestimmter Katalysatoren herzustellen.

So wird in der DE-A-3 739 549 ein Verfahren zur Herstellung (cyclo)aliphatischer Uretdione beschrieben, bei dem (cyclo)aliphatische Diisocyanate als Ausgangsstoffe und Pyridine als Katalysatoren eingesetzt werden.

Aus Richter und Ulrich, Synthesis 1975, 463, ist ein Verfahren bekannt, bei dem Benzylisocyanate in Gegenwart von 1,2-Dimethylimidazol als Katalysator umgesetzt werden, wobei jedoch ca. 20 % Isocyanurat anfallen. Für eine Reihe von Anwendungen ist die Anwesenheit von Isocyanuraten unerwünscht, da diese trifunktionell sind und zur Vernetzung neigen.

Auch bei Noack und Schwetlick, Z. Chem. 26 (1), 117 (1986), ist dieser Nachteil der Bildung von Isocyanuraten zu finden. Bei der Reaktion von T80, einem Gemisch aus 80 Gew.-% 2,4- und 20 Gew.-% 2,6-Toluylendiisocyanat mit 1-Methyl-, 1,2-Dimethyl- oder 1-Butylimidazol als Katalysatoren in einer Mischung aus Methylethylketon und Cyclohexan, werden nur höchstens 51 % Dimerausbeuten erhalten.

In der polnischen Patentbeschreibung PL-100148 wird die Reaktion von aromatischen Diisocyanaten mit 1-Allyl-2-methylimidazol als Katalysator beschrieben. Hierbei beträgt jedoch die Dimerausbeute lediglich 50 %.

Der Erfindung lag daher die Aufgabe zugrunde, ein Verfahren zur Herstellung von Uretdiongruppen aufweisenden Polyisocyanaten zu finden, das die Bildung von Isocyanuraten weitestgehend einschränkt.

Die Aufgabe wird erfindungsgemäß dadurch gelöst, daß monomere aromatische Diisocyanate in Anwesenheit eines Katalysators umgesetzt werden, wobei als Katalysator Imidazole oder Benzimidazole der allgemeinen Formeln I oder II 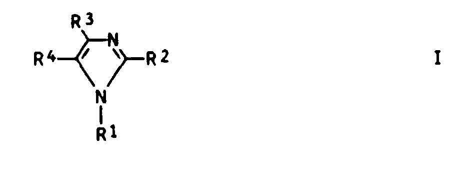 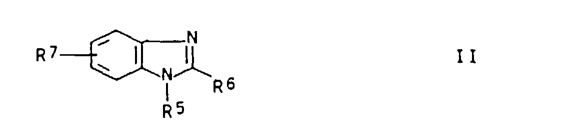 oder deren Mischungen eingesetzt werden, wobei
- R¹, R⁵: unabhängig voneinander Wasserstoffatome, C₁- bis C₁₆-Alkylgruppen, Arylgruppe, Alkylaryl- oder Aralkylgruppen, wobei die Alkylgruppen 1 bis 10 C-Atome aufweisen können, Amino-, Hydroxy- oder Mercaptogruppen, Aminoalkyl-, Alkylamino-, Alkoxy-, Oxyalkyl-, Alkylthio- oder Thioalkylgruppen mit C₁- bis C₁₆-Alkylgruppen oder Halogenatome bedeuten,
- R², R³, R⁴, R⁶, R⁷: die gleiche Bedeutung wie R¹ und R⁵ haben oder eine Nitrogruppe sind, wobei R² und R⁶ keine Wasserstoffatome bedeuten
und wobei folgende Verbindungen ausgeschlossen ist:
1,2-Dimethylimidazol.

Bevorzugte Ausführungsformen des erfindungsgemäßen Verfahrens sind den Unteransprüchen zu entnehmen.

Weiterhin zeigte sich, daß der Gehalt an Isocyanuraten sowohl beim Einsatz von wasserhaltigem als auch von trockenem Lösungsmittel unter 2 % liegt.

Als monomere aromatische Diisocyanate eignen sich beispielsweise
1,5-Naphthalindiisocyanat, 4,4'-Diphenyldiisocyanat,
1,4-Phenylendiisocyanat sowie vorzugsweise 2,6-Toluylendiisocyanat;
besonders bevorzugt sind 2,4-Toluylendiisocyanat sowie 4,4'-Diphenylmethandiisocyanat.

Die Reaktion kann in Substanz oder in Gegenwart eines inerten organischen Lösungsmittels stattfinden, wobei letztere Ausführungsform bevorzugt ist. Besonders bevorzugt ist die Durchführung der Reaktion in einem inerten organischen Lösungsmittel mit einem Wassergehalt von 50 bis 500 ppm. Als Lösungsmittel sind hierfür vorzugsweise Toluol und n-Hexan zu nennen. Von großer Bedeutung ist der Trocknungsgrad des Lösungsmittels. Bei der Verwendung von handelsüblichem Toluol (Wassergehalt ca. 300 bis 500 ppm) oder handelsüblichem n-Hexan (Wassergehalt ca. 200 bis 300 ppm) werden Dimerausbeuten von über 90 % erhalten, das Produkt ist aber leicht durch Harnstoffe verunreinigt. Wird dagegen trockenes Lösungsmittel verwendet (Wassergehalt < 100 ppm), sind die Ausbeuten an Uretdionen zwar etwas geringer, aber das Produkt ist nahezu frei von Harnstoffen. Man erhält jedoch sowohl beim Einsatz eines wasserhaltigen als auch eines trockenen Lösungsmittels einen Gehalt an Isocyanuraten von weniger als 2 %.

Das Gewichtsverhältnis des Lösungsmittels zum Diisocyanat liegt im Bereich von 0,1:1 bis 10:1. Bevorzugt wird ein Verhältnis von 0,3:1 bis 3:1 eingesetzt.

Zu dem gelösten Diisocyanat wird bei Temperaturen von -10°C bis 80°C, bevorzugt bei 20°C bis 50°C unter Rühren der Katalysator, ebenfalls in Lösung, zugesetzt.

Die als Katalysatoren erfindungsgemäß einzusetzenden Imidazole I oder Benzimidazole II können jeweils alleine oder in Mischungen verwendet werden. Weiterhin sind Mischungen aus I und II möglich. Als besonders geeignet haben sich 1,2,4,5-Tetramethyl-, 1-n-Butyl-2,4,5-trimethyl- und 1-Benzyl-2-methylimidazol erwiesen. Die Katalysatoren werden in Mengen von 0,01 bis 50 Gew.-%, bevorzugt 0,05 bis 1 Gew.-%, bezogen auf das Diisocyanat, eingesetzt. Das Gewichtsverhältnis des Lösungsmittels zum Imidazol bzw. Benzimidazol liegt im Bereich von 1:1 bis 100:1, vorzugsweise von 10:1 bis 60:1.

Das entstandene Umsetzungsprodukt wird gewaschen und getrocknet.

Die mit der Erfindung erzielten Vorteile bestehen insbesondere darin, daß der Gehalt an unerwünschtem Isocyanurat unter 2 % liegt, und daß man bei der Reaktion in Lösung über den Trocknungsgrad des Lösungsmittels die Reinheit und die Ausbeute der Uretdione beeinflussen kann.

Die so hergestellten Uretdiongruppen aufweisenden Polyisocyanate können zur Polyurethanherstellung verwendet werden.

### Beispiele: Umsetzung von 2,4-Toluylendiisocyanat (TDI) und 4,4'-Diphenylmethandiisocyanat (MDI)

500 g monomeres aromatisches Diisocyanat und 200 g Lösungsmittel wurden unter Rühren mit 2,5 g des entsprechenden Imidazols, gelöst in 100 g Lösungsmittel, bei Temperaturen von 20 °C im Fall des TDI's bzw. 50°C im Fall des MDI's, versetzt. Bei dieser Temperatur wurde 5 Stunden gerührt und die entstandene Suspension 16 Stunden lang bei Raumtemperatur stehengelassen. Das nun feste Produkt wurde zweimal mit je 500 g Lösungsmittel gewaschen und im Vakuum getrocknet.

Über die als Katalysatoren verwendeten Imidazole, die Lösungsmittel und die Ausbeuten an Uretdionen gibt die Tabelle Aufschluß.

**Tabelle**

| Beispiel | Diisocyanat | Imidazol | Lösungsmittel | Ausbeute an Uretdion [%] |
|---|---|---|---|---|
| 1 | TDI | 1,2,4,5-Tetramethylimidazol | Toluol | 96 |
| 2 | TDI | " | Toluol trocken | 81 |
| 3 | TDI | " | n-Hexan | 95 |
| 4V | TDI | 1-Allyl-2-methylimidazol | Toluol | 50 |
| 5 | TDI | 1-n-Butyl-2,4,5-trimethylimidazol | Toluol | 95 |
| 6 | TDI | " | Toluol trocken | 60 |
| 7 | TDI | 1-Benzyl-2-methylimidazol | Toluol | 93 |
| 8 | TDI | " | Toluol trocken | 44 |
| 9 | 80 Gew.-% 2,4-Toluylendiisocyanat + 20 Gew.-% 2,6-Toluylendiisocyanat | 1,2,4,5, Tetramethylimidazol | Toluol | 60 |
| 10V | " | 1-Methylimidazol | Methylethylketon/Cyclohexan | 46 |
| 11 | MDI | 1,2,4,5-Tetramethylimidazol | Toluol | 83 |
| 4V: Vergleichsbeispiel in Anlehnung an PL-100148 unter Verwendung von reinem 2,4-Toluylendiisocyanat | | | | |
| 10V: Vergleichsbeispiel nach Noack und Schwetlick, Z. Chem. 26 (1), 117 (1986) | | | | |

## Patentansprüche

1. Verfahren zur Herstellung von Uretdiongruppen aufweisenden Polyisocyanaten durch Reaktion von monomeren aromatischen Diisocyanaten in Anwesenheit eines Katalysators, dadurch gekennzeichnet, daß als Katalysator Imidazole oder Benzimidazole der allgemeinen Formeln I oder II 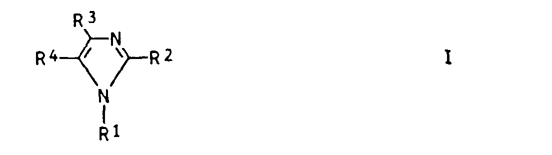 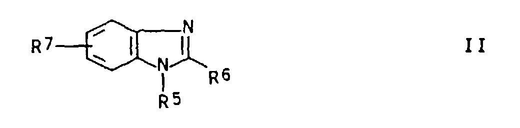 oder deren Mischungen eingesetzt werden, wobei
R¹, R⁵ unabhängig voneinander Wasserstoffatome, C₁- bis C₁₆-Alkylgruppen, Arylgruppe, Alkylaryl- oder Aralkylgruppen, wobei die Alkylgruppen 1 bis 10 C-Atome aufweisen können, Amino-, Hydroxy- oder Mercaptogruppen, Aminoalkyl-, Alkylamino-, Alkoxy-, Oxyalkyl-, Alkylthio- oder Thioalkylgruppen mit C₁- bis C₁₆-Alkylgruppen oder Halogenatome bedeuten,
R², R³, R⁴, R⁶, R⁷ die gleiche Bedeutung wie R¹ und R⁵ haben oder eine Nitrogruppe sind, wobei R² und R⁶ keine Wasserstoffatome bedeuten
und wobei folgende Verbindungen ausgeschlossen ist:
1,2-Dimethylimidazol.

2. Verfahren nach Anspruch 1, dadurch gekennzeichnet, daß als Imidazole I 1,2,4,5-Tetramethylimidazol oder 1-n-Butyl-2,4-5-trimethylimidazol oder 1-Benzyl-2-methylimidazol eingesetzt werden.

3. Verfahren nach den Ansprüchen 1 bis 2, dadurch gekennzeichnet, daß man die Reaktion in einem inerten organischen Lösungsmittel mit einem Wassergehalt von 50 bis 500 ppm durchführt.

## Claims

1. A process for preparing polyisocyanates containing uretdione groups by reaction of monomeric aromatic diisocyanates in the presence of a catalyst, wherein the catalyst used is an imidazole or benzimidazole of the formula I or II 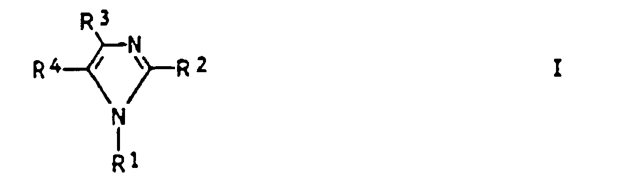 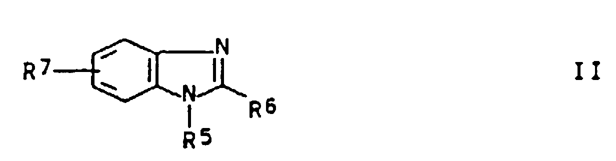 or a mixture thereof, where
R¹, R⁵ are, independently of one another, hydrogen atoms, C₁-C₁₆-alkyl groups, aryl groups, alkylaryl or aralkyl groups in which the alkyl groups may have from 1 to 10 carbon atoms, amino, hydroxy or mercapto groups, aminoalkyl, alkylamino, alkoxy, oxyalkyl, alkylthio or thioalkyl groups having C₁-C₁₆-alkyl groups or halogen atoms,
R², R³, R⁴, R⁶, R⁷ are as defined for R¹ and R⁵ or are a nitro group, where R² and R⁶ are not hydrogen atoms,
with the exception of the following compounds:
1,2-dimethylimidazole.

2. A process as claimed in claim 1, wherein the imidazole I is 1,2,4,5-tetramethylimidazole or 1-n-butyl-2,4,5-trimethylimidazole or 1-benzyl-2-methylimidazole.

3. A process as claimed in claim 1 or 2, wherein the reaction is carried out in an inert organic solvent having a water content of from 50 to 500 ppm.

## Revendications

1. Procédé pour la préparation de polyisocyanates présentant des groupes uretdione, par réaction de diisocyanates monomères aromatiques en présence d'un catalyseur, caractérisé en ce qu'on met en oeuvre comme catalyseur des imidazoles ou des benzimidazoles de formule générale I ou II 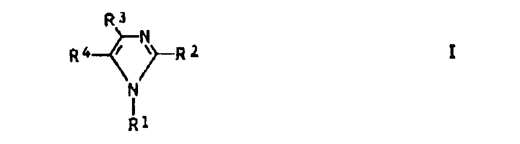 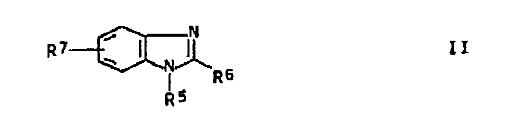 ou leurs mélanges, où
R¹, R⁵, signifient, indépendamment l'un de l'autre, des atomes d'hydrogène, des groupes alkyle en C₁ à C₁₆, des groupes aryle, des groupes alkylaryle ou aralkyle, les groupes alkyle pouvant présenter 1 à 10 atomes de carbone, des groupes amino, des groupes hydroxy ou des groupes mercapto, des groupes aminoalkyle, des groupes alkylamino, des groupes alkoxy, des groupes oxyalkyle, des groupes alkylthio ou des groupes thioalkyle comprenant des groupes alkyle en C₁ à C₁₆ ou des atomes d'halogène
R², R³, R⁴, R⁶, R⁷ ont la même signification que R¹ et R⁵ ou sont un groupe nitro, R² et R⁶ ne signifiant pas des atomes d'hydrogène,
et le composé suivant étant exclu :
1,2-diméthylimidazole.

2. Procédé selon la revendication 1, caractérisé en ce qu'on met en oeuvre comme imidazoles I, du 1,2,4,5-tétraméthylimidazole ou du 1-n-butyl-2,4,5-triméthylimidazole ou du 1-benzyl-2-méthylimidazole.

3. Procédé selon les revendications 1 à 2, caractérisé en ce qu'on effectue la réaction dans un solvant organique inerte présentant une teneur en eau de 50 à 500 ppm.
